Europäisches Patentamt

European Patent Office

Office européen des brevets

(19)

(11) Publication number: 0 239 878
A1

# (12) EUROPEAN PATENT APPLICATION

(21) Application number: 87103953.3

(22) Date of filing: 07.10.83

(51) Int. Cl.⁴: C12Q 1/32 ,
//(C12Q1/32,C12R1:41)

(30) Priority: 08.10.82 US 433503

(43) Date of publication of application:
07.10.87 Bulletin 87/41

(60) Publication number of the earlier application in
accordance with Art.76 EPC: 0 108 508

(84) Designated Contracting States:
AT BE CH DE FR GB IT LI LU NL SE

(71) Applicant: STATE OF OREGON, by and
through the Oregon State Board of Higher
Education for Oregon State University
P.O. Box 3175
Eugene Oregon 97403(US)

(72) Inventor: Cantrell,Michael A.,
421,S.W.8th
Corvallis,Oregon 97333(US)
Inventor: Haugland,Richard A.,
961 N.W.Hayes,Apt.18
Corvallis,Oregon 97330(US)
Inventor: Evans,Harold J.,
2939 N.W.Mulkey
Corvallis,Oregon 97330(US)

(74) Representative: Brown, John David et al
FORRESTER & BOEHMERT
Widenmayerstrasse 4/I
D-8000 München 22(DE)

(54) Process for identifying a colony of Rhizobium japonicum.

(57) A process for identifying a colony of Rhizobium japonicum having hydrogen-uptake activity from a group of colonies, some of which do not have hydrogen-uptake activity, comprising:

replicating colonies of the group onto a surface of a porous, sterile support material;

incubating cells on the surface under conditions that derepress their hydrogenase;

treating the support material with a methylene blue indicator solution containing inhibitors of endogenous respiration;

maintaining the support material with attached colonies in an $H_2$ atmosphere whereupon colonies with hydrogen uptake activity reduce the methylene blue to its leuco form.

EP 0 239 878 A1

## "PROCESS FOR IDENTIFYING A COLONY OF RHIZOBIUM JAPONICUM"

### SUMMARY OF THE INVENTION

The present invention relates to nitrogen fixation by leguminous plants. More specifically, it relates to pure strains of nitrogen-fixing microorganisms that contain insert DNA to facilitate the oxidation of $H_2$ produced as a byproduct of nitrogen fixation and thereby increase the amount of energy available for the nitrogen fixation process.

To facilitate the fixation of nitrogen in leguminous plants, seeds are inoculated with desirable strains of nitrogen-fixing microorganisms. A variety of techniques are used, including the mixing of cultures with the soil where plants are to be grown. More often, seeds are coated with a medium that includes the desired nitrogen-fixing microorganism. When seeds with such a coating thereon germinate, the seedling plants are directly exposed to the microorganism.

Nitrogenases from legume nodules and all known sources catalyze an ATP-dependent reduction of not only $N_2$ to $NH_4$, but also protons to $H_2$. This $H_2$ production results in an inefficient use of the energy provided by the organism to the $N_2$-fixing process. Most $N_2$-fixing, free-living microorganisms, but a minority of strains of Rhizobium, possess the capacity for synthesis of an $H_2$-recycling system which oxidizes the $H_2$ produced during $N_2$ fixation, thus recapturing some of the energy expended during $H_2$ evolution. [Evans, H. J., Purohit, K., Cantrell, M. A., Eisbrenner, G., Russell, S. A., Hanus, F. J., and Lepo, J. E. 1981, Current Perspectives in Nitrogen Fixation, ed. Gibson, A. H. and Newton, W. E. (Elsevier/North Holland, New York, NY), 84-96].

Dixon [Dixon, R. O. D. 1978. Biochimie 60, 233-236.] pointed out several potential advantages of $H_2$-oxidizing capability to $N_2$-fixing organisms. Subsequent investigations have shown that an active $H_2$-oxidizing system in R. japonicum can increase the ATP content of bacteroid cells and also provide a mechanism for additional protection of bacteroid nitrogenase from $O_2$ inactivation [Emerich, D. W., Ruiz-Argüeso, Ching, T. M., and Evans, H. J. 1979. J. Bacteriol. 137, 153-160.]

In glasshouse and field experiments, soybean plants inoculated with groups of hydrogenase positive (Hup+) strains of R. japonicum were reported to contain higher percentages of N in their tissues and seeds than plants inoculated with groups of hydrogenase negative (Hup−) strains [Albrecht, S. L., Maier, R. J., Hanus, F. J., Russell, S. A., Emerich, D. W., and Evans, H. J. 1979, Science 203, 1255-1257], [Hanus, F. J., Albrecht, S. L., Zablotowicz, R. M., Emerich, D. W., Russell, S. A., and Evans, H. J. 1981, Agron. J. 73, 368-372]. The strains of R. japonicum with highly active $H_2$-oxidizing capacities have been shown to grow as chemolithotrophs using $H_2$ and $CO_2$ as their sources of energy and carbon, respectively [Hanus, F. J., Maier, R. J., & Evans, H. J. (1979) Proc. Natl. Acad. Sci. USA 76, 1788-1792.].

$H_2$ is oxidized by the hydrogenase enzyme and $CO_2$ is fixed by a RuBP carboxylase [Lepo, J. E., Hanus, F. J., and Evans, H. J. (1980) J. Bacteriol. 141, 664-670]. Strains which possess such chemolithotrophic capacity may have an increased chance of survival in the soil where there is a limitation of fixed carbon substrates.

Highly active hydrogen-oxidizing systems have been found only in about 25% of R. japonicum strains and in no strain of R. trifolii or R. meliloti [Evans, H. J., Purohit, K., Cantrell, M. A., Eisbrenner, G., Russell, S. A., Hanus, F. J., & Lepo, J. E. (1981) Current Perspectives in Nitrogen Fixation, eds. Gibson, A. H. & Newton, W. E., (Elsevier/North Holland, New York, NY) 84-96.]. Most strains of R. leguminosarum are Hup− and with a few exceptions the Hup+ strains of this species recycle only a small proportion of the $H_2$ produced by the nitrogenase reaction [Nelson, L. M. and Salminen, S. O. (1982) J. Bacteriol. 151, 989-995]. It would be beneficial if Hup− strains of any one of the several species of Rhizobium could be modified to Hup+, e.g. by a transfer of the genes for the Hup phenotype from highly active Hup+ strains. Beneficial effects of the transfer of genes for the Hup phenotype could include not only the ability to utilize $H_2$ per se but also the ability to grow chemolithotrophically.

At least some of the determinants for the Hup phenotype have been shown to be plasmid-borne and transmissible in Alcaligenes eutrophus [Friedrich, B., Hogrefe, C., & Schlegel, H. G. (1981) J. Bacteriol. 147, 198-205] and in R. leguminosarum [Brewin, N. J., DeJong, T. M., Phillips, D. A. and Johnston, A. W. B. (1980) Nature 288, 77-79]. In both R. leguminosarum and A. eutrophus, the Hup phenotype was carried on large, naturally occurring plasmids which must carry many other genes. The plasmid carrying Hup determinants in R. leguminosarum also was shown to carry determinants for nodulation specificity and for $N_2$ fixation. The host range for this plasmid may be limited [Brewin, N. J., DeJong, T. M., Phillips, D. A. & Johnston, A. W. B. (1980) Nature 28 , 77-79], and recipients of this plasmid may only be able to form effective nodules on pea plants.

Techniques for measurement of $H_2$ uptake have been described [Hanus, F. J., Carter, K. R. & Evans, H. J. (1980) Methods Enzymol. 69, 731-739.] [Bethlenfalvay, G. J. & Phillips, D. A. (1979) Plant Physiol. 63, 816-820.]. Colonies of Azotobacter chroococcum have been screened for expression of hydrogenase activity by observations of differences in rates of reduction of methylene blue by Hup+ and Hup- Azotobacter colonies [Postgate, J. R., Partridge, C. D. P., Robson, R. L., Simpson, F. G., & Yates, M. G. (1982) J. Gen. Microbiol. 128, 905-908.]. But none of these methods relates to the isolation of Hup+ Rhizobium colonies from a large number of colonies.

A procedure for the introduction of genetic information for hydrogen-uptake systems into a broad host range, transmissible DNA replicon has now been developed. A definitive method of screening large numbers of bacterial colonies for the necessary isolation of strains carrying determinants for hydrogenase expression also has now been discovered. Two products of these procedures, pHUl and pHU2 DNA's, contain hydrogen-uptake genes from Rhizobium japonicum strain 122 DES and have been transferred from Escherichia coli to the Hup- R. japonicum mutant strains PJl7nal and PJl8nal whereupon they converted free-living cells of these strains, most likely by genetic complementation in the first instance and by recombination in the second instance, to a Hup+ phenotype. Both pHUl and pHU2 can give bacteroids of PJl7nal, occurring in soybean nodules, sufficient hydrogen-uptake activity to utilize all hydrogen gas produced in the nodules. These products and other products constructed by the described procedure may be used to introduce genetic information for a hydrogen-uptake system into strains of Rhizobium currently lacking such systems. Expression of the introduced genetic information of Hup- recipient Rhizobium strains will increase the amount of energy available for nitrogen fixation.

## DETAILED DESCRIPTION

The process of this invention employs recombinant DNA techniques in conjunction with a screening method to allow production and isolation of recombinant replicons containing DNA which codes for genes specific for the hydrogen-uptake phenotype. These replicons are referred to herein as pHU DNA's. Specific examples of such replicons are the cosmids pHUl and pHU2 described below. Section I below is a detailed description of a process for production and isolation of pHU DNA. Section II is a detailed description of recombinant cosmids made by the process.

The following definitions may be useful in understanding the terminology of this disclosure:

Replicon. - A genetic element which is capable of independent replication. This may include a plasmid, cosmid, or bacteriophage DNA.

Hup+. - A designation for the phenotype of any organism having the ability to show uptake of hydrogen gas - synonymous with hydrogen gas oxidation and hydrogen uptake.

Hup-. - A designation for the phenotype of any organism which does not have the ability to show hydrogen uptake.

hup. - A designation for a hydrogen uptake gene.

HU DNA. - DNA which contains hup genes or portions of hup genes.

pHU DNA. - A replicon produced by recombinant DNA techniques, which contains HU DNA.

## I. Production and Isolation of pHU DNA

An object of the present process is to isolate and produce DNA which can be used to provide hydrogen uptake activity to Hup- Rhizobium strains and in particular DNA which returns hydrogen uptake activity to certain specific Hup- test strains of R. japonicum. Such DNA may come from any organism which contains hup genes capable of either showing complementation to the Hup- test strains R. japonicum or showing recombination with these strains of R. japonicum, thus converting them to a Hup+ phenotype. The vector DNA to which the Hup gene-containing DNA (the insert DNA) is attached may be any replicon which can be introduced and maintained in R. japonicum.

A process according to this invention includes a number of steps, including one or more of the following:

A. Growth of bacterial strains and isolation of DNA.

B. Construction of insert DNA/vector DNA recombinants.

C. Introduction of recombinant DNA into recipient strains.

D. Identification of recipients containing pHU DNA.

E. Isolation of pHU DNA.

Step D is of particular interest in that it involves a procedure to distinguish colonies of the Hup phenotype from thousands of colonies of R. japonicum. Colonies are grown on plates of any standard culture medium and then replicated onto discs (for example, filter paper discs) and incubated under conditions which will derepress the hydrogen-uptake system. After an appropriate period of time, the discs are soaked in a solution containing methylene blue and inhibitors of endogenous respiration. The concentration of inhibitors should be sufficient to block methylene blue dye reduction via endogenous respiration. When the discs are then incubated under a stream of $H_2$, only colonies with an active hydrogenase ($Hup^+$) will show $H_2$-dependent methylene blue dye reduction as evidenced by conversion of the methylene blue in that area of the disc to a colorless form.

## A. Growth of bacterial strains and isolation of DNA

Strains of E. coli, unless otherwise indicated are grown on LB medium at 32°C [Kahn, M., et al (1979) Methods Enzymol. 68, 268-280, incorporated herein by reference]. R. japonicum are routinely cultured in broth or on agar plates using the yeast extract mannitol medium described by Vincent [Vincent, J. M. (1970) A Manual for the Practical Study of Root-Nodule Bacteria (Blackwell, Oxford, UK) 59-60, incorporated herein by reference] or the hydrogen uptake medium described by Maier, et al (Maier, R. J., et al. (1978) Prose; Natl. Acad. Sci., USA 75, 3258-3262], adjusted to pH 7.0 and modified by adding 0.2 g $KH_2PO_4 \cdot H_2O$ and 0.03 g.
$NaH_2PO_4 \cdot H_2O$ per liter instead of 0.15 g
$NaH_2PO_4 \cdot H_2O$. Strains of E. coli and R. japonicum used are listed in Table I. The cosmid, pLAFRI, was produced by Friedman et al (Friedman, A. M. Long, S. R., Brown, S. E., Buikema, W. J., & Ausubel, F. M. (1982) Gene 18, 289-296, incorporated herein by reference] from the plasmid, pRK290, which was produced by G. Ditta, S. Stranfield, D. Corbin, and D. R. Helinski [(1980), Proc. Natl. Acad. Sci, USA 77, 7347-7351, incorporated herein by reference] and is a broad host range cloning vehicle. The nal$^r$ derivatives of SR, PJI7 and PJI8 listed in table I are spontaneous nalidixic acid-resistant mutants obtained by standard techniques.

Table I    Bacterial Strains

| Strain | Relevant Characteristics | Source or Reference |
|---|---|---|
| **E. coli** | | |
| HB101 | recA⁻, hsdR, hsdM, pro, leu, Str$^r$ | 1 |
| HB101 (pLAFR1) | contains pLAFR1 | 2 |
| HB101 (pRK2013) | contains pRK2013 | 3 |
| BHB 2688 | N205, recA, (λimm434, cIts, b2, red3, Eam4, Sam7)/λ | 4 |
| BHB2690 | N205, recA⁻, (λimm434, cIts, b2, red3, Dam15, Sam7)/λ | 4 |
| **R. japonicum** | | |
| 122 DES | Hup⁺, derivative of USDA 122 | 5 |
| SR | Hup⁺, Str$^r$, Kan$^r$, derivative of 122 DES | 6 |
| SRnal | Nal$^r$ derivative of SR | 7 |
| PJ17 | Hup⁻, Str$^r$, Kan$^r$, derivative of SR | 8 |
| PJ17nal | Nal$^r$ derivative of PJ17 | 9 |
| PJ18 | Hup⁻, Str$^r$, Kan$^r$, derivative of SR | 8 |
| PJ18nal | Nal$^r$ derivative of PJ18 | 10 |

1. Boyer, H. B., et al. (1969) J. Mol. Biol. 41, 459-472; obtainable from ATCC 39195 and 39196.

2. Friedman, A. M., et al. (1982) Gene 18, 289-296.

3. Ditta, G., et al. (1980) Proc. Natl. Acad. Sci. USA 77, 7347-7351.

4. Hohn, B. (1979) Methods Enzymol. 68, 299-309.

5. Ruiz-Argüeso, T., et al. (1979) Arch. Microbiol. 121, 199-206.

6. Maier, R. J., et al. (1978) Nature 276, 494-495.

7. Cantrell, M. A., et al. (1982) unpublished, attached.

8. Lepo, J. E., et al. (1981) J. Bacteriol. 146, 614-620.

9. ATCC 39194

10. ATCC 39193

Each of the writings mentioned in the above notes is incorporated herein by reference.

Large scale preparations of cosmid DNA from E. coli and R. japonicum are obtained by the procedure of Cantrell, Hickok and Evans [(1982) Arch. Microbiol. 131, 102-106., incorporated herein by reference] For use in preparation of the clone bank, pLAFR1 DNA from E. coli is further purified by two CsCl/ethidium bromide gradient centrifugations in 49% (w/w) CsCl and 0.35 mg/ml ethidium bromide in a buffer solution at pH 8.0 containing 50 mM Tris-HCl, 20 mM EDTA (TE buffer). The initial refractive index of the gradient solution is 1.3910. Samples are centrifuged in a Beckman VTi65 rotor to equilibrium (12-16 hr) at 55,000 rpm. Covalently-closed circular DNA recovered from the second gradient centrifugation is diluted with TE buffer and pelleted by centrifugation in a Beckman SW60 rotor. The pelleted DNA is resuspended in 0.37 ml of a buffer containing 6 mM Tris-HCl, pH 7.4 10 mM NaCl, 0.1 mM EDTA (DNA storage buffer), extracted repeatedly with isoamyl alcohol and precipitated with ethanol. After drying the precipitate by evacuation at room temperature, it is redissolved and stored in 0.25 ml DNA storage buffer.

Small scale isolations of cosmid DNA from E. coli for use in restriction analyses are performed by use of a cleared lysate procedure [Kahn, M., Kolter, R., Thomas, C., Figurski, D., Meyer, R., Remaut, E., & Helinski, D. R. (1979) Methods Enzymol. 68, 271-272, incorporated herein by reference]. Total genomic DNA from 500 ml cultures of R. japonicum grown in HUM broth medium to an optical density at 540 nm of approximately 1.0 is isolated by a previously described procedure [Haugland, R. A., & Verma, D. P. S. (1981) J. Mol. Appl. Genet. 1, 205-217, incorporated herein by reference] with the following modification. After the spooled, ethanol-precipitated DNA is dried, it is redissolved in 5 ml of a pH 7.0 buffer solution containing 150 mM NaCl and 15 mM sodium citrate (1 $\times$ SSC). A 1 mg/ml solution of RNase A in the same buffer is preheated 15 minutes at 80° and then added to the DNA solution to a final concentration of 50 $\mu$g/ml. After a 30 minute incubation at 37°, the DNA is again spooled onto a glass rod from an overlay of two volumes of cold ethanol and subsequently treated as referred to above.

## B. Construction of insert DNA/vector DNA recombinants

R. japonicum DNA which is to be used as the insert DNA (HU DNA) is digested with the restriction enzyme EcoRI and size fractionated by the following procedure. R. japonicum 122 DES total DNA (200 $\mu$g) is incubated at 37° with 20 $\mu$l of EcoRI (Bethesda Research Laboratories, Gaithersburg, MD, U.S.A.; 10 units/$\mu$l) for periods ranging from 20 to 115 minutes in 100 mM Tris-HCl, pH 7.2, 50 mM NaCl, 5 mM MgCl$_2$ and 2 mM $\beta$-mercaptoethanol. Aliquots from each EcoRI digestion time point are subjected to agarose gel electrophoresis by the method of Meyers et al [Meyers, J. A., Sanchez, D., Elwell, L. P., & Falkow, S. (1976) J. Bacteriol. 127, 1529-1537, incorporated herein by reference] with modifications of Haugland & Verma [Haugland, R. A., & Verman, D. P. S. (1981) J. Mol. Appl. Genet. 1, 205-207, incorporated herein by reference] to identify samples with average sizes of approximately 30 kb, 22 kb and 15 kb. Approximately 50 $\mu$g of DNA falling into each of these three classes are then pooled and extracted first with equal volumes of phenol (equilibrated with 100 mM Tris, pH 8.5) and then twice with double volumes of ether equilibrated as above. The DNA is then precipitated with 2 volumes of ethanol, incubated more than 30 minutes at -20°C, pelleted by centrifugation for 15 minutes in an Eppendorf microfuge, washed with cold 80% ethanol, recentrifuged as above, dried under vacuum and finally redissolved in 0.7 ml of a buffer containing 20 mM Tris-HCl, 10 mM EDTA, 50 mM NaCl, pH 8.0 (sucrose gradient buffer). The sample is heated for 10 minutes at 65°C and then layered on a sucrose gradient and centrifuged using the conditions described by Ditta et al [Ditta, G., Stanfield, S., Corbin, D., and Helinski, D. R. (1980) Proc. Natl. Acad. Sci. USA 77, 7347-7351, incorporated herein by reference]. Aliquots from 0.7 ml fractions taken from the bottom of the gradient are then subjected to agarose gel electrophoresis as described above to identify the fractions containing DNA in the size range of 12 to 30 kb. Fractions containing DNA in this size range are pooled and dialyzed at 4°C against several changes of 1/10-strength sucrose gradient buffer over a period of 24 hours. The dialyzed sample is then precipitated with two volumes of ethanol, pelleted by centrifugation at 15,000 RPM for 20 minutes in a Sorvall SS-34 rotor, washed with cold 80% ethanol, and recentrifuged as above, dried under vacuum and finally redissolved in 0.4 ml of DNA storage buffer.

The pLAFR1 DNA is digested with EcoRI under the conditions described above with the exception that EcoRI is added at a ratio of 5 units per $\mu$g of DNA, the mixture is incubated 90 minutes at 37°C, an additional 5 units of EcoRI per $\mu$g of DNA is added, the mixture is incubated an additional 90 minutes at 37°C, and the reaction is terminated by adding a volume of 0.25 M Na$_2$EDTA (pH 8.0) equal to 6% of the reaction volume to give a final concentration of 8 mM EDTA. The reaction mixture is extracted 2 times with equal volumes of redistilled phenol, then 3 times with equal volumes of ether, and traces of remaining ether are then removed by evaporation with a stream of N$_2$.

Ligation is performed as follows: 20 μg of pooled, partially digested I2-30 kb fractions of R. japonicum DNA are added per μg of EcoRI-digested pLAFRI, the mixture is ethanol precipitated as described above, and the precipitate is redissolved in ligation buffer (66 mM Tris-HCl, pH 7.6, 6.6 mM MgCl₂). The resultant mixture of EcoRI-digested pLAFRI and partially digested R. japonicum DNA is then ligated at concentrations of 0.05 and I.0 mg/ml, respectively, in the presence of 700 U/ml T4 ligase (Bethesda Research Laboratories), 66 mM Tris-HCl, pH 7.6, 6.6 mM MgCl₂, I0 mM dithiothreitol and 66 μM ATP for I8 hours at I2°C.


## C. Introduction of recombinant DNA into recipient strains

The recombinant DNA ligation mixture is packaged with an extract containing phage lambda head and tail components and the resultant packaged cosmids are adsorbed onto E. coli strain HBI0I.

Packaging extracts are prepared and DNA is packaged with the following modifications of the procedure of Blattner et al. [Blattner, F. R., Blechl, A. E., Denniston-Thompson, K., Faber, H. E., Richards, J. E., Slightom, J. L., Tucher, P. W., & Smithies, O. (I978) Science 202, I279-I284, incorporated herein by reference] using the lambda lysogenic strains of E. coli BHB2688 and BHB2690 (SEE Table I). These lambda lysogens are each grown as 500 ml cultures in LB medium in a 2 l. flask at 32°C to approximately 3 × I0⁸ cells per ml. The cultures are heated in a water bath to 45°C, then maintained at that temperature for 30 minutes with constant agitation. they are then placed on a shaker at 37° for I hour and then chilled on ice. All succeeding production of the extracts is at 4°C.

Production of the BHB2688 extract: Cells from three 500 ml cultures of BHB2688 are each centrifuged I0 minutes at 9000 rpm in a Sorvall GSA rotor and the pellets are resuspended in a total of 3 ml of a buffer containing I0% sucrose, 50 mM Tris-HCl, pH 7.4. A total of I50 μg of highly purified lysozyme (Sigma) is then added in a volume of 75 μl of 0.25 M Tris-HCl, pH 7.4. The sample is mixed gently and frozen in liquid N₂. It is then thawed on ice, 400 μl of the M I buffer of Blattner et al. is added, it is incubated 30-45 minutes at 4°C, and centrifuged 35 minutes at 28,000 rpm in a Beckman type 30 rotor. The BHB2688 extract consists of aliquots of the supernatant stored at -70°C.

Production of the BHB2690 extract: Cells from one culture of 500 ml of BHB2690 are centrifuged I0 minutes at 9000 rpm in a Sorvall GSA rotor and the pellet is resuspended in 3.I ml of the buffer A of Blattner et al. The suspension is sonicated as described by Blattner, et al. and centrifuged 5 minutes at 7000 rpm in a Sorvall SS-34 rotor. The BHB2690 extract consists of aliquots of the supernatant stored at -70°C.

Packaging of DNA and Transfer in E. coli. DNA packaging is initiated by addition of the following in the order indicated: 7 μl of the buffer A of Blattner et al, 3 μl of ligated DNA, I μl of the MI buffer of Blattner et al, 5 μl of BHB2690 extract and 5 μl of BHB2688 extract. After incubation for I hour at 23°C, a drop of CHCl₃ is added, the mixture is serially diluted, and transduction with strain HBI0I is performed as described by Hohn [Hohn, B. (I979) Methods Enzymol. 68, 299-309, incorporated herein by reference].

Selection for tetracycline-resistant transductants on LB plates with 20 μg/ml of tetracycline results in the growth of a large number of colonies which constitute a gene bank. During generation of the gene bank which lead to isolation of pHUI and pHU2, tetracycline resistant transductants were obtained at a frequency of 7.8 × I0⁵ per μg of vector DNA. The resultant gene bank contained more than 40,000 clones. Analysis of 24 of these clones chosen at random has shown that 83% contain insert DNA with an average molecular size of 22.6 kb.

PJI7nal is an R. japonicum revertible Hup⁻ mutant, ATCC 39I94. This biologically pure culture is available from the permanent collection of the American Type Culture Collection, Rockville, Maryland, U.S.A. Colonies from the clone bank were conjugated en masse with PJI7nal by use of the triparental mating system of Ditta et al [Ditta, G., Stanfield, S., Corbin, D., & Helinski, D. R. (I980) Proc. Natl. Acad. Sci. USA 77, 7347-735I, incorporated herein by reference]. In this procedure, cultures of E. coli containing recombinant cosmids and E. coli containing the mobilization helper plasmid pRK20I3, both grown to early log phase (approximately 0.4 absorbance units at 600 nm) in LB medium are each concentrated by centrifugation at 8000 rpm in a Sorvall SS-34 rotor and resuspended in 0.05 vol. of YEM medium. A culture of R. japonicumrecipient cells, grown to late log phase (approximately 0.5 absorbance units at 540 nm) in YEM medium is likewise concentrated. Aliquots of 0.I ml of each of these three cell suspensions (approximately 5 × I0⁸ R. japonicum cells and approximately 5 × I0⁷ of each of the two E. coli cell types) are then spread together on a YEM medium agar plate at pH 7.0, excess liquid is removed by evaporation under a sterile, laminar-flow hood, and the cells are then incubated at 29°C for 3 to 4 days. After this time, the cells are scraped off the plate and suspended in 8 ml of the mineral salts solution used for the HUM

medium containing 0.01% Tween 80. Dilutions ranging from $10^{-1}$ to $10^{-5}$ are plated on either YEM or HUM medium with 100 $\mu$g/ml tetracycline and 100 $\mu$g/ml nalidixic acid. Recipient colonies containing pHU DNA are then identified from plates containing up to 3000 transconjugant colonies.

## D. Identification of recipients containing pHU DNA

Recipient colonies which have received DNA from the clone bank (transconjugants) are replicated onto sterile filter paper discs (Whatman 541) which are then placed colony side up on plates of Repaske's medium [Repaske, R., & Repaske, C. (1976) App. Env. Microbiol. 32, 585-591; Repaske, R., & Mayer, R. (1976) Appl. Env. Microbiol. 32, 592-597, both incorporated herein by reference].

Cells on the filters are then derepressed by incubation for 4 to 5 days in an atmosphere which initially contains 5% $H_2$, 5% $CO_2$, approximately 0.7% $O_2$, and the remainder $N_2$. $O_2$ is consumed such that levels of $O_2$ gradually decrease to less than 0.1% of the gas volume during incubation and are subsequently maintained at about 0.2% by additions of $O_2$. In cases where many $Hup^+$ colonies are present during the derepression period, $H_2$ levels also decrease as determined by gas chromatography and are subsequently maintained at approximately 5% by additions of $H_2$.

Filters with depressed colonies are removed from the Repaske plates and soaked in 0.8 ml of a solution containing 200 mM iodoacetic acid (Sigma Chemical Co., St. Louis, MO, U.S.A.), 200 mM malonic acid, 10 mM methylene blue (Nutritional Biochemical Co.), 50 mM $KH_2PO_4$, 2.5 mM $MgCl_2$ adjusted to pH 5.6 with KOH. After approximately 15 min, the plates are tilted slightly and excess solution is removed from the sides of the filters with a Pasteur pipette. An additional 45 min in air is allowed for the dye/inhibitor solution to equilibrate with the colonies, and the plates with filters and attached colonies, are transferred to a Plexiglass tray (inside dimensions: $29 \times 39 \times 3$cm) with an outer well filled with water. A Plexiglass cover with side walls that fit into the outer well of the tray is used to form a partial seal against entry of air into the tray. The cover also contains two portals to permit controlled entry and exit of cylinder gases.

$N_2$ is flushed through the chamber for 15 minutes to reduce the $O_2$ concentration over the filters. The chamber is placed in a fume hood for safety purposes and $H_2$ (passed through a catalytic $O_2$ purifier - Engelhard Industries, Inc.) is then introduced at a flow rate of approximately 150 ml. per minute. Both gases are passed through $H_2O$ before entering the Plexiglass chamber in order to reduce evaporation from the filters. Colonies with $H_2$ uptake activity reduce methylene blue to its leuco form within 0.5 to 3 hours while $Hup^-$ colonies do not reduce methylene blue during periods of 20 hours and more. $Hup^+$ colonies are found amongst the population of $Hup^-$ recipients at frequencies of approximately $10^{-2}$ to $10^{-3}$. The $Hup^+$ colonies are then isolated from the master plates and pHU cosmid DNA is isolated as described below.

## E. Isolation of pHU DNA

Colonies showing methylene blue reduction activity in the initial screening are picked from the original plates from which the filter replicas were taken and streaked for single colonies on HUM medium agar plates containing 100 $\mu$g/ml each of tetracycline and nalidixic acid. When colonies on these plates have grown to maximum size (10-15 days), the plates are replicated onto filter paper discs and the portions of the colonies transferred are derepressed and subjected to the above-described methylene blue reduction screening procedure for the identification of recipients containing pHU DNA. Isolated colonies identified as having dye reducing activity in this second screening are picked from the master plates and used to inoculate 200 ml YEM broth cultures containing 45 $\mu$g/ml tetracycline. The cultures are grown to an optical density of approximately 0.2 absorbance units at 540 nm and the cells then harvested and subjected to the plasmid isolation procedure of Cantrell et al. [(1982) Arch. Microbiol. 131, 102-106]. Partially purified cosmid DNA obtained by this procedure is then used to transform E. coli HB101 cells by the procedure of Davis et al [Davis, R. W., Botstein, D., & Roth, J. R. (1980) A Manual for Genetic Engineering: Advanced Bacterial Genetics (Cold Spring Harbor Laboratory, Cold Spring Harbor, NY) 140-141, incorporated herein by reference] with the modification that cryogenically stored, competent E. coli cells, prepared by the method of Morrison [Morrison, D. A. (1979) Methods Enzymol. 68, 326-331, incorporated herein by reference] are used as recipients. The transformed E. coli are selected on LB medium with 20 $\mu$g/ml tetracycline.

The resultant transformants are used to purify pHU cosmid DNA by standard techniques. For example, the above-described procedures for growth of bacterial strains and isolation of DNA are used and the procedure of Guerry, LeBlanc, and Falkow [J. Bacteriol. (1973) 116, 1064-1066, incorporated herein by reference] followed by CsCl/EtBr gradient centrifugation is used.

## II. Characteristics of pHUl and pHU2

Two cosmids isolated by the above procedures are pHUl and pHU2. pHUl is obtainable from E-scherichia coli HB101 (pHUl), ATCC 39195. pHU2 is obtainable from Escherichia coli HB101 (pHU2), ATCC 39196. These biologically pure cultures are available from the permanent collection of the American Type Culture Collection, Rockville, MD, U.S.A. The replicons are unique because they contain HU DNA which is attached by recombinant DNA techniques to vector DNA. The specific vector DNA used (pLAFRI) is not itself new. Other vector DNA may be similarly usable so long as it can be easily introduced and maintained in R. japonicum and a bacterium such as E. coli, commonly used in genetic laboratories. The HU DNA is the insert DNa which produces the Hup⁺ character in the recipient test strains as described below. The HU DNA may come from any organism which contains hup genes. The characteristics listed below should not be construed as limiting, nor need the functional characteristics be specifically associated with DNA fragments having the molecular sizes listed.

## A. Physical characteristics of pHUl and pHU2

Both the cosmids pHUl and pHU2 are approximately 47 kb in molecular size and thus have a molecular weight of approximately $3 \times 10^7$. The approximate molecular sizes in kilobase pairs of DNA fragments produced from restriction endonuclease of pHUl and pHU2 with EcoRI and a combination of EcoRI and BglII are given in Table II. Fragments designated by asterisks in the table occur in the cosmid vector pLAFRI and for this reason are not fragments of HU DNA. Results given in the table were obtained by digesting pHUl and pHU2 for two hours at 37° with 5.5 units EcoRI (Bethesda Research Laboratories, Inc.) per µg DNA and/or 5 units BglII (Boerhinger Mannheim, Inc.) per µg DNA. The buffer used for all digestions contains 50 mM Tris, pH 8.0, 10 mM MgCl₂, 50 mM NaCl and 2 mM β-mercaptoethanol. Following restriction endonuclease digestions, the DNA's were electrophoresed in a 0.8% agarose gel made up in a buffer containing 89 mM Tris, 2.5 mM EDTA and 89 mM boric acid, pH 8.3. Additional fragments produced in the restriction digests with sizes less than 0.4 kilobase pairs would not have been detected in these analyses.

The DNA used as a source of HU DNA (R. japonicum 122 DES DNA) was originally digested with the restriction enzyme EcoRI. As a result, the HU DNA present in pHUl and pHU2 could be easily cleaved from the vector, pLARFI, by partial digestions with EcoRI by procedures well known in the art. Commonly used procedures would then allow joining of the HU DNA with different replicons so that pHU insert DNA can be transferred to many species of bacteria. Because pLAFRI is a wide host range vector, pHUl and pHU2 can be inserted in multiple species of bacteria whereby, for example, pHU insert DNA can be easily maintained in E. coli, transferred from E. coli to R. japonicum, and maintained in the R. japonicum.

## TABLE II

### Restriction Fragment Sizes in Kb Pairs of pHU1 and pHU2 DNA's

| pHU1 | | pHU2 |
| --- | --- | --- |
| Digestion with EcoRI | Digestion with EcoRI + BglII | Digestion with EcoRI |
| 21.6* | 19.0* | 21.6* |
| 13.0 | 7.6 | 13.0 |
| 5.0 | 5.0 | 4.8 |
| 2.9 | 2.9 | 2.9 |
| 2.3 | 2.7 | 2.3 |
| 1.55 | 1.64* | 2.2 |
| 0.60 | 1.55 (doublet) | 0.60 |
| | 1.51 | |
| | 1.09* | |
| | 0.95 | |
| | 0.68 | |
| | 0.60 | |

B. Functional characteristics of pHUI and pHU2

Conjugation of HBI0I containing pHUI or pHU2 with R. japonicum PJI7nal by the procedures described above results in introduction of the cosmids into PJI7nal. Derepressed cells of R. japonicumstrain PJI7nal (see Section I, Part D above for derepression procedure) containing pHUI or pHU2 DNA's exhibit both methylene blue and $O_2$-dependent $H_2$ uptake activity. The aforementioned activities occur as a result of genetic complementation of a mutated DNA sequence present within strain PJI7nal by insert DNA sequences occurring with the HUI or HU2 DNa's and are comparable with the activities demonstrated by derepressed free-living cells of the Hup+ R. japonicum strain I22 DES. Derepressed cells of PJI7nal containing the vector pLAFRI show no $H_2$-uptake activity.

$O_2$-dependent $H_2$-uptake activity is measured by the following method. R. japonicum cells are grown to an absorbance (540 nm) of 0.25 in 8 ml of YEM medium (containing 50 μg/ml of tetracycline when strains with plasmids are used). Cells are harvested by centrifugation and cell pellets resuspended in 0.2 ml of HUM medium. The suspensions are spread on plates of Repaske's medium and derepressed for 4 days under the gas mixture described for the methylene blue colony screen. Cells are removed from the plates and suspended in 5 ml of 50 mM $KH_2PO_4$ and 2.5 mM $MgCl_2$ buffer adjusted to pH 7.0 with KOH. The suspensions are diluted I0-fold in the above buffer and assayed amperometrically for rates $O_2$-dependent $H_2$ uptake by the method of Hanus, Carter and Evans[(I980) Methods Enzymol. 69, 73I-739, incorporated herein by reference].

Using the growth conditions and procedures described below, soybeans infected with PJI7nal containing pHUI and pHU2 DNA's can form nodules which demonstrate no $H_2$ evolution. In a nodulation test which was done as described below, soybean plants infected with PJI7nal containing pHUI or pHU2 formed nodules which demonstrated no $H_2$ evolution. Acetylene reduction activities by these nodules and those formed by strain I22 DES after similar inoculations and growth of soybeans are comparable. The pHU insert DNA does not interfere with the ability of PJI7nal to effectively nodulate soybean plants and likely will not interfere with the ability of any recipient Rhizobium species to effectively nodulate its natural plant host.

For plant tests, soybean seed [Glycine max (L.) Merr., cultivar Wilkin] are surface disinfected by the following procedure. Seeds are soaked in 70% ethanol for I0-20 seconds, rinsed 5-8 times with sterile distilled water, incubated 3 minutes in a 2% solution of sodium hypochlorite, then rinsed 5-8 times more with sterile distilled water and germinated on plates of 0.8% agar, containing one-half strength N-free nutrient solution [Harper, J. E. & Nicholas, J. C. (I976) Physiol. Plant 38, 24-28, incorporated herein by reference] to which is added 3 mM $CaSO_4$. After 48 hours, seedlings are immersed for 20 minutes in 5 day-old YEM broth cultures (with 45 $\mu g/ml$ tetracycline for transconjugants) of desired strains of R. japonicum. The inoculated seedlings are transferred to a sterile mixture of equal parts sand and vermiculite in 250 ml flasks. Seedlings are protected by cotton plugs and placed in a growth cabinet (I6 hr day - 8 hr night cycle) at an irradiance of about 250 $\mu E.m^{-1}.sec^{-1}$. The day and night temperatures are maintained near 28°c and 23°C, respectively. Plants receive sufficient sterile one-half strength N-free nutrient solution [Harper, J. E., & Nicholas, J. C. (I976) Physiol. Plant 38, 24-28, incorporated herein by reference] to maintain a moist medium without allowing free solution in the flasks. Plants are harvested 30 days after germination and nodules are assayed for rates of $C_2H_2$ reduction and $H_2$ evolution and for bacteroid hydrogenase activity by procedures used by Lepo et al [Lepo, J. E., Hickok. R. E., Cantrell, M. A., Russell, S. A., & Evans, H. J. (I98I) J. Bacteriol. I46, 6I4-620, incorporated herein by reference].

PJI8 nal is another R. japonicum revertible Hup⁻ mutant, ATCC 39I93. This biologically pure culture is available from the permanent collection of the American Type Culture Collection, Rockville, MD, U.S.A. Conjugation of HBI0I (pHUI) or HBI0I (pHU2) with PJI8nal allows introduction of the cosmids into PJI8nal. Methylene blue-dependent hydrogen-uptake activity is exhibited by colonies containing either pHUI or pHU2 at frequencies of approximately I-3 $\times$ $10^{-3}$.

These frequencies of appearance of Hup⁺ colonies are calculated by dividing the number of Hup⁺ tetracycline resistant colonies by the total number of tetracycline resistant colonies.

The strains PJI7nal and PJI8nal lack the ability to grow chemolithotrophically only because they are Hup⁻ due to a single point mutation. The strain from which they were derived, SR, had the ability to grow chemolithotrophically. A number of PJI7nal transconjugants which were converted to Hup⁺ were identified by their ability to grow chemolithotrophically after introduction of pHU cosmids. The cosmids pHUI and pHU2 were not analyzed in this manner, but since they convert PJI7nal to a Hup⁺ character, they should also convert PJI7nal into a chemolithotroph.

While we have described and given examples of preferred embodiments of our inventions, it will be apparent to those skilled in the art that changes and modifications may be made without departing from our inventions in their broader aspects. We therefore intend the appended claims to cover all such changes and modifications as fall within the true spirit and scope of our inventions.

The features disclosed in the foregoing description, in the following claims and/or in the accompanying drawings may, both separately and in any combination thereof, be material for realising the invention in diverse forms thereof.

## Claims

I. A process for identifying a colony of Rhizobium japonicum having hydrogen-uptake activity from a group of colonies, some of which do not have hydrogen-uptake activity, comprising:

replicating colonies of the group onto a surface of a porous, sterile support material;

incubating cells on the surface under conditions that derepress their hydrogenase;

treating the support material with a methylene blue indicator solution containing inhibutors of endogenous respiration;

maintaining the support material with attached colonies in a $H_2$ atmosphere whereupon colonies with hydrogen uptake activity reduce the methylene blue to its leuco form.

2. The process of claim I wherein:

the support material comprises filter paper;

during the incubating, the filter paper is placed colony side up on plates of Repaske's medium;

the incubating occurs in a closed container wherein the atmosphere is initially about 5% $H_2$, 5% $CO_2$, 0.7% $O_2$, and the remainder $N_2$, the level of $O_2$ being allowed to decrease to below 0.1% during incubating;

the level of $O_2$ thereafter is maintained at about 0.2% by additions of $O_2$;

the methylene blue indicator solution contains 200 mM iodoacetic acid, 200 mM malonic acid, 10 mM methylene blue, 50 mM $KH_2PO_4$, 2.5 mM $MgCl_2$, and an amount of KOH sufficient to adjust the pH to 5.6.

| | DOCUMENTS CONSIDERED TO BE RELEVANT | | EP 87103953.3 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | METHODS IN ENZYMOLOGY, vol. 69, 1980, New York, London, Toronto, Sydney, San Francisco<br><br>F.J.HANUS et al. "Techniques for Measurement of Hydrogen Evolution by Nodules"<br>pages 731-739<br><br>* Page 731, lines 1-20 *<br><br>-- | 1 | C 12 Q 1/32<br>(C 12 Q 1/32<br>C 12 R 1:41) |
| D,A | PLANT PHYSIOLOGY, vol. 63, no. 4, April 1979<br><br>G.J.BETHLENFALVAY AND D.A. PHILLIPS "Variation in Nitrogenase and Hydrogenase Activity of Alaska Pea Root Nodules"<br>pages 816-820<br><br>* Abstract *<br><br>-- | 1 | |
| D,A | THE JOURNAL OF GENERAL MICROBIOLOGY, vol. 128, 1982<br><br>J.R.POSTGATE et al. "A Method for Screening for Hydrogenase Negative Mutants of Azotobacter chroococcum"<br>pages 905-908<br><br>* Pages 906,907 *<br><br>-- | 1 | TECHNICAL FIELDS SEARCHED (Int. Cl.4)<br><br>C 12 Q<br>C 12 N<br>G 01 N 33/00 |
| A | US - A - 4 026 767 (SHIH et al.)<br><br>* Abstract *<br><br>-- | 1 | |

The present search report has been drawn up for all claims

| Place of search<br>VIENNA | Date of completion of the search<br>09-07-1987 | Examiner<br>SCHNASS |
|---|---|---|

Application number

European Patent
Office

**EUROPEAN SEARCH REPORT**

| DOCUMENTS CONSIDERED TO BE RELEVANT | | | EP 87103953.3 |
|---|---|---|---|
| Category | Citation of document with indication, where appropriate, of relevant passages | Relevant to claim | CLASSIFICATION OF THE APPLICATION (Int. Cl 4) |
| A | DR. OTTO-ALBRECHT NEUMÜLLER "Römpps Chemie-Lexikon", 7th edition, part 4:"M-Pk", 1974 FRANCKH'SCHE VERLAGSHANDLUNG, Stuttgart pages 2150-2151 * Page 2151, left col., lines 24-27 * -- | 1 | |
| A | SARTORIUS LABORFILTRATION MIKRO-BIOLOGIE, 1979 pages 18,19 * Totality * ---- | 1 | |
| | | | TECHNICAL FIELDS SEARCHED (Int. Cl 4) |
| | Refund: 100 % | | |

The present search report has been drawn up for all claims

| Place of search | Date of completion of the search | Examiner |
|---|---|---|
| VIENNA | 09-07-1987 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82